# FASCICULE DE BREVET EUROPEEN

(11) **EP 4 132 391 B1**
(45) Date de publication et mention de la délivrance du brevet: **20.03.2024**
(21) Numéro de dépôt: 21722106.8
(22) Date de dépôt: 08.04.2021
(51) Int. Cl.: A61B 17/68, A61B 17/84, A61C 8/00, A61B 17/86

(54) **IMPLANT D'ANCRAGE OSSEUX À STABILISATION CORTICALE**
KORTIKAL STABILISIERTES KNOCHENVERANKERUNGSIMPLANTAT
CORTICALLY STABILIZED BONE ANCHORING IMPLANT

(30) Priorité: 09.04.2020 FR 2003579
(43) Date de publication de la demande: 15.02.2023
(73) Titulaire: Lock-In SA, 1180 Rolle (CH)
(72) Inventeur: LACAZE, Guillaume, 1278 LA RIPPE (CH)
(74) Mandataire: Debay, Damien
(86) Numéro de dépôt international: PCT/EP2021/059195
(87) Numéro de publication internationale: WO 2021/204951

(56) Documents cités:
- DE-A1- 19 646 307
- DE-U1- 29 619 163
- FR-A1- 2 965 473
- US-A- 5 004 421
- US-A- 5 087 199
- US-A1- 2012 265 258
- US-A1- 2013 053 902
- US-A1- 2018 263 669

## Description

### DOMAINE TECHNIQUE ET OBJET DE L'INVENTION

La présente invention se rapporte au domaine des implants osseux destinés à des applications dentaires, orthopédiques, chirurgicales ou ostéoplastiques, tels que des vis orthopédiques seules ou avec des plaques, des implants dentaires ou ligamentaires pour des articulations tels que par exemple les hanches, les coudes, les chevilles, les épaules et les genoux, ou spinaux rachidiens par exemple pour les vertèbres. Ces domaines d'application sont donnés à titre d'exemple et ne sont pas restrictifs quant à la portée de la présente invention.

Plus spécifiquement l'invention concerne un implant osseux dont l'implantation dans l'os poreux est extrêmement stable.

### ETAT DE LA TECHNIQUE

Un implant d'ancrage osseux est généralement constitué d'un corps de forme allongée destiné à être implanté dans un logement formé dans un tissu osseux, tel que l'os de la mâchoire pour une application dentaire ou dans une vertèbre par exemple. Il est important que l'implant d'ancrage osseux puisse être introduit facilement dans le tissu osseux, sans créer de dommages, et que le dispositif d'ancrage à l'intérieur du tissu osseux soit stable. En effet, les dispositifs d'implant d'ancrage osseux actuels ne permettent pas un ancrage sans engendrer des fissures ou des dommages plus importants que ne le nécessite la taille du dispositif lui-même dans le tissu osseux, de plus il est nécessaire que la fixation dans l'implant osseux soit fiable et extrêmement stable, car de nombreuses techniques thérapeutiques reposent aujourd'hui sur la croissance osseuse qui nécessite généralement que les dispositifs ancrés dans le tissu osseux restent le plus immobile possible.

En outre, il est aussi nécessaire que l'implantation dans le tissu osseux soit facile à réaliser pour éviter tout risque de mauvais positionnement de l'implant osseux, qui pourrait notamment être dû à une difficulté de positionnement ou d'implantation dans l'os.

De plus, en cas de chute, de choc ou d'accident, il est important que l'implant reste en place dans le tissu osseux, c'est-à-dire qu'il ne se déplace pas à travers l'os. Pour cela, une très forte stabilité de l'implant est nécessaire.

L'état de la technique comprend le document de brevet EP2603163 B1, qui décrit un implant endo-osseux à ancrage amélioré apte à être implanté dans un tissu osseux et comportant un dispositif de fixation comprenant une partie dite d'accrochage dans le tissu osseux, et une partie dite d'expansion, ces deux parties étant mobiles l'une par rapport à l'autre. L'invention mentionnée dans ce brevet comprend également des moyens de liaison mécanique coopérants, disposés d'une part sur la partie d'accrochage et d'autre part sur la partie d'expansion, tels que la mobilité relative des deux parties comprend au moins un degré de liberté et tels qu'un déplacement relatif desdites deux parties provoque un élargissement de la partie d'accrochage, ledit élargissement provoquant l'accrochage de la partie d'accrochage dans le tissu osseux. L'implant osseux décrit dans ce brevet trouve particulièrement application dans le domaine dentaire.

Cependant, une telle solution présente des inconvénients car l'implant osseux bien qu'il soit immobilisé en rotation et en translation dans le tissu, il présente le risque de bouger, notamment de reculer lors d'un choc.

L'invention vise donc à résoudre ces inconvénients en proposant un implant osseux apte à être implanté et immobilisé dans le tissu osseux de façon extrêmement stable.

Le document de brevet DE 196 46 307 A1 divulgue un implant d'ancrage osseux à stabilisation corticale apte à être implanté dans un tissu osseux, comprenant un manchon expansible ayant un filetage interne et un filetage externe et un corps de vis présentant un profil extérieur complémentaire au profil intérieur du manchon expansible et un filetage extérieur. L'implant est apte à passer d'une position repliée de repos à une position déployée.

### PRESENTATION GENERALE DE L'INVENTION

La présente invention a donc pour objet de pallier les inconvénients de l'art antérieur en proposant un implant d'ancrage osseux, ci-après dénommé implant osseux, facilement implantable dans le tissu osseux et stable.

Pour parvenir à ce résultat, la présente invention concerne un implant d'ancrage osseux à stabilisation corticale apte à être implanté dans un tissu osseux, comprenant :
Un manchon expansible s'étendant entre une portion proximale possédant un premier diamètre intérieur, et une portion distale possédant un second diamètre intérieur inférieur audit premier diamètre intérieur, ces deux portions définissant un axe longitudinal (L) et lesdits premier et second diamètres intérieur définissant un profil intérieur dudit manchon expansible, et comprenant, d'une part, au moins un premier filetage à l'intérieur du manchon expansible et, d'autre part, au moins un second filetage à l'extérieur du manchon expansible,
Un corps de vis s'étendant entre une portion proximale et une portion distale et présentant, d'une part, le long dudit axe longitudinal (L), un profil extérieur complémentaire au profil intérieur dudit manchon expansible et, d'autre part, au moins un filetage extérieur dont le pas de vis est inversé par rapport audit second filetage extérieur du manchon expansible,
L'implant étant apte à passer d'une position repliée de repos à une position déployée par l'actionnement desdits filetages inversés en provoquant la pénétration de la vis dans le manchon expansible et engendrant l'expansion dudit manchon expansible par déformation, grâce au fait que le diamètre extérieur de la vis est supérieur, au moins sur une portion distale, audit second diamètre intérieur du manchon expansible, par au moins un rétrécissement,

En position déployée de l'implant, le second diamètre intérieur du manchon expansible étant supérieur ou égal au premier diamètre proximal du manchon,

Le manchon possédant une forme cylindrique ou une portion tronconique obtenue par ladite expansion,

L'implant possédant, à proximité de sa portion proximale en position déployée, une portion tronconique proximale s'évasant en direction de la portion proximale et formée :
Soit par le profil extérieur dudit manchon,
Soit par le profil extérieur dudit corps de vis,
Soit par la complémentarité de forme des profils extérieurs du manchon et du corps de vis, en position déployée,
Ladite portion tronconique proximale possédant un filetage externe d'ancrage osseux sur sa périphérie.

Selon une particularité, ledit au moins un rétrécissement est situé, par rapport à la portion proximale et selon l'axe longitudinal (L), à une distance déterminée en fonction de la profondeur, dans le tissu osseux, à laquelle ladite expansion est souhaitée.

Selon une particularité, la vis est configurée pour s'implanter dans le tissu osseux en s'enfonçant à l'intérieur du manchon expansible, sa portion proximale comprenant le filetage externe d'ancrage osseux est adaptée à être implantée dans le tissu osseux et présente un profil externe tronconique sur sa portion proximale dont l'angle d'ouverture du cône est inversé par rapport à celui de la portion tronconique du manchon expansible en position déployée.

Selon une autre particularité, la vis est configurée pour s'implanter dans le tissu osseux en s'enfonçant à l'intérieur du manchon expansible, sa portion proximale comprenant le filetage externe d'ancrage osseux est adaptée à être implantée dans le tissu osseux et présente un profil externe cylindrique sur sa portion proximale. Selon une autre particularité, ladite portion tronconique de la portion proximale du manchon expansible et ledit profil externe tronconique de la vis sont positionnés en regard l'un de l'autre.

Selon une autre particularité, l'angle de la portion tronconique de la portion proximale du manchon expansible est supérieur à l'angle du profil externe tronconique de la vis pour permettre un évasement plus grand et améliorer la stabilité primaire.

Selon une autre particularité, la vis comprend au moins un marqueur de distance pour visualiser le moment où le vissage de la vis dans le manchon expansible doit être réalisé dans le sens opposé au vissage du manchon expansible dans le tissu osseux. Selon une autre particularité, la hauteur de filet du second filetage extérieur du manchon expansible est supérieure à celle du filetage mécanique du premier filetage à l'intérieur du manchon expansible et du filetage extérieur de la vis.

Selon une autre particularité, la portion distale du manchon expansible présente une portion tronconique comprenant un filetage avec une âme conique permettant au manchon expansible de s'enfoncer profondément dans l'os.

Selon une autre particularité, la portion distale comporte des encoches auto-taraudantes.

Selon une autre particularité, le manchon expansible comporte des fentes longitudinales débouchantes s'étendant jusqu'à sa portion distale.

Selon une autre particularité, il y a autant d'encoches auto-taraudantes que de fentes longitudinales débouchantes.

Selon une autre particularité, le manchon expansible comporte des fentes longitudinales non débouchantes.

Selon une autre particularité, la vis comprend à sa portion distale une pointe comportant au moins une goujure arrière à bord coupant dont l'angle par rapport à un axe longitudinal (L) définit par les deux extrémités s'étendant entre la portion proximale et la portion distale du manchon expansible, est déterminé en fonction du sens de rotation de la vis lors du dévissage depuis la position déployée vers la position de repos, pour fraiser l'os lors de l'extraction de l'implant osseux.

### PRESENTATION DES FIGURES

D'autres caractéristiques et avantages de l'invention apparaîtront à la lecture de la description détaillée des modes de réalisation de l'invention, donnés à titre d'exemple uniquement, et en référence aux dessins qui montrent :
[Fig. 1a], [Fig. 1b] et [Fig. 2] représentent une vue détaillée des éléments qui composent l'implant osseux selon l'invention.
[Fig. 3a] représente une vue détaillée du manchon expansible avant l'expansion selon l'invention.
[Fig. 3b] représente une vue détaillée du manchon expansible après expansion selon l'invention.
[Fig. 3c] représente une vue détaillée de la portion tronconique proximale en position déployée formée par le profil extérieur du manchon, selon l'invention.
[Fig. 4a] et [Fig. 4b] représentent un schéma d'une coupe transversale de l'intérieur de l'implant osseux, avant l'expansion du manchon expansible, selon l'invention.
[Fig. 5] représente une vue détaillée de la portion tronconique proximale en position déployée formée par le profil extérieur de la vis, selon l'invention.
[Fig. 6] et [Fig. 7] représentent un schéma de l'implant osseux en position expandue, selon l'invention.
[Fig. 8] et [Fig. 9] représentent une vue du manchon expansible en position expandue selon l'invention.
[Fig. 10a], [Fig. 10b] et [Fig. 11] représentent une vue de l'intérieur de l'implant osseux en position expandue selon l'invention.
[Fig. 12] représente un schéma de la pointe de la vis, selon l'invention.
[Fig. 13] représente une vue détaillée de la portion tronconique proximale en position déployée formée par les profils extérieur de la vis et du manchon, selon l'invention

### DESCRIPTION DETAILLE D'UNE FORME DE REALISATION DE L'INVENTION

Divers modes de réalisation de l'invention sont décrits ci-dessous notamment en référence aux figures illustratives et non limitatives.

La présente demande concerne l'implantation d'un implant osseux dans un tissu osseux.

On notera ici que l'on désigne par le terme « implantation » le fait d'introduire l'implant osseux dans le tissu osseux, généralement par vissage. L'implantation proposée dans la présente demande désigne une introduction suffisamment solide et stable de l'implant osseux pour assurer un bon maintien de cet implant osseux dans le tissu osseux.

Par ailleurs, le terme « tissu(s) osseux » désigne généralement tous types d'os, qu'il s'agisse d'os compact (comme par exemple l'os cortical ou le périoste) ou d'os spongieux (mou, poreux), car le système d'implant osseux de la présente demande est implantable dans tout type de tissu osseux.

En outre, les termes utilisés ne doivent pas être interpréter dans leur acceptation générale mais plutôt à la lumière des considérations fonctionnelles détaillées dans la présente demande.

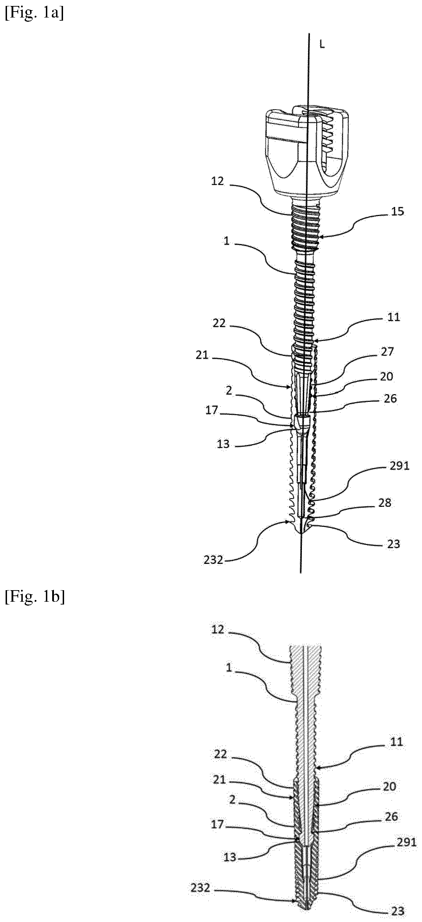

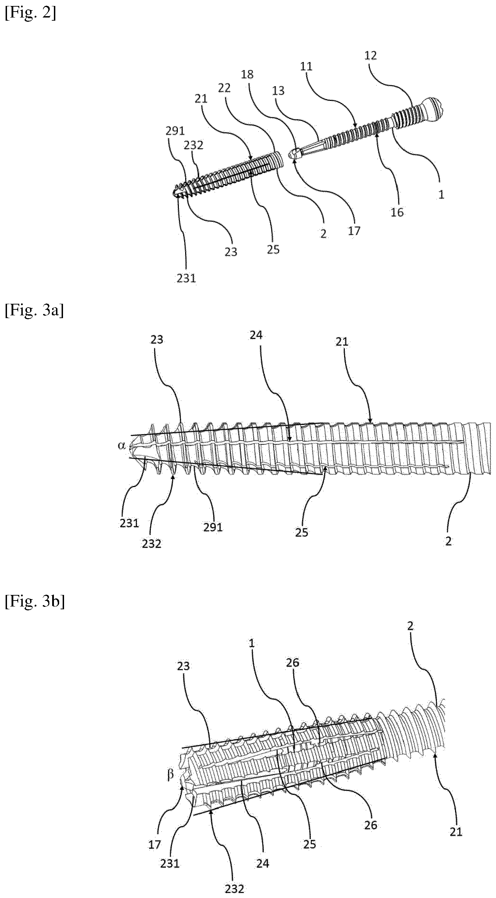

[Fig. 1a], [Fig. 1b] et [Fig. 2] sont des exemples de réalisation illustratifs et non limitatifs de l'implant osseux.

Comme par exemple représenté sur [Fig. 1a], [Fig. 1b] et [Fig. 2], un implant osseux apte à être implanté dans un tissu osseux comprend : un manchon expansible (2) s'étendant entre une portion proximale (22) et une portion distale (23), les extrémités de ces deux portions définissant un axe longitudinal (L), et comprenant, d'une part, au moins un premier filetage (20) à l'intérieur du manchon expansible (2) et, d'autre part, au moins un second filetage (21) à l'extérieur du manchon expansible (2).

Les termes « proximal » et « distal » désignent dans la présente demande, respectivement, la partie où le dispositif d'implantation est tenu pour permettre son implantation dans le tissu osseux, et la partie qui est implantée en premier dans le tissu osseux (à l'opposé de la portion proximale).

Les termes « portions » proximale et distale désignent dans la présente demande les parties situées à proximité des extrémités distales et proximales.

Dans la présente demande, le terme manchon expansible (2) désigne généralement un cylindre généralisé (generalized cylinder en anglais) creux.

Dans certains modes de réalisation, l'implant osseux comprend aussi une vis (1) s'étendant entre une portion proximale (12) et une portion distale (13) sur un axe colinéaire à l'axe (L) et présentant, d'une part, le long dudit axe longitudinal (L), un profil extérieur complémentaire au profil intérieur dudit manchon expansible (2) et, d'autre part, au moins un filetage (11) extérieur dont le pas de vis est inversé par rapport audit second filetage extérieur (21) du manchon expansible (2).

Il est à noter que la portion proximale (12) de la vis (1) est directement implantée dans l'os cortical.

Dans certains modes de réalisation, l'extrémité proximale de la vis (1) comprend un moyen d'actionnement permettant de visser la vis (1), ledit moyen d'actionnement comprenant une structure de toute forme souhaitable par le praticien selon l'utilisation qui en sera faite, comme par exemple représenté sur [Fig. 1b]. Le moyen d'actionnement étant par exemple un trou à six pans ou un torx ou un cruciforme ou tout autre moyen d'actionnement, et l'extrémité proximale de la vis (1) pourra avoir diverses formes en fonction de la destination souhaitable pour l'implant d'ancrage osseux (tête de fixation de barre d'ostéosynthèse polyaxiale ou non, ou fixation de plaque ou tout autre dispositif).

Dans certains modes de réalisation, la vis (1) comprend une canule traversant la vis (1) pour permettre au praticien d'injecter par exemple du ciment, s'il estime cela nécessaire.

Il est aussi à noter que l'implant osseux est réalisé en titane ou en acier inoxydable médical implantable ou en polyétheréthercétone (PEEK) ou en polyéthercétonecétone (PEKK) ou en tout autre matériau dont l'homme du métier pourra déterminer l'adéquation en fonction de ses propriétés mécaniques, physico-chimiques et de sa biocompatibilité.

Dans certains modes de réalisation, la vis (1) comprend une canule traversant la vis (1) pour permettre au praticien d'injecter par exemple du ciment, s'il estime cela nécessaire.

Dans certains modes de réalisation, le second filetage (21) à l'extérieur du manchon expansible (2) permet l'ancrage osseux. Le terme « ancrage osseux » utilisé dans la présente demande désigne de façon générale divers types de dispositifs comprenant au moins un élément destiné à pénétrer le tissu osseux, selon un trajet rectiligne, sous l'action d'une poussée généralement exercée sous la forme de frappes, d'impacts, ou de vissages répétés. Il est connu qu'un filetage d'ancrage osseux possède une hauteur de filet généralement supérieure à celle d'un filetage mécanique pour assurer un meilleur ancrage. En outre, un filetage d'ancrage osseux est généralement différent d'un filetage mécanique et l'Homme du métier sait, qu'en fonction du type d'os et de l'application souhaitée, il est possible de faire varier le diamètre de l'âme, le pas de vis et la hauteur de fil et la présente demande couvre ces divers modes de réalisation. La hauteur de filet du second filetage extérieur (21) du manchon expansible (2) est supérieure à celle du filetage mécanique du premier filetage (20) à l'intérieur du manchon expansible (2) et du filetage (11) extérieur de la vis (1). Ainsi, le second filetage (21) comprend des bords plus hauts que le premier filetage (20) pour permettre à l'implant osseux de rentrer et de s'ancrer dans l'os.

De plus, dans certains modes de réalisation, certains filets mécaniques, comme par exemple les filets trapézoïdaux, offrent moins de résistance ce qui facilite la pénétration de la vis (1) dans le manchon expansible (2) ancré dans l'os. Le filet trapézoïdal permet également de répartir la charge importante de tissu osseux en compression contre l'implant osseux.

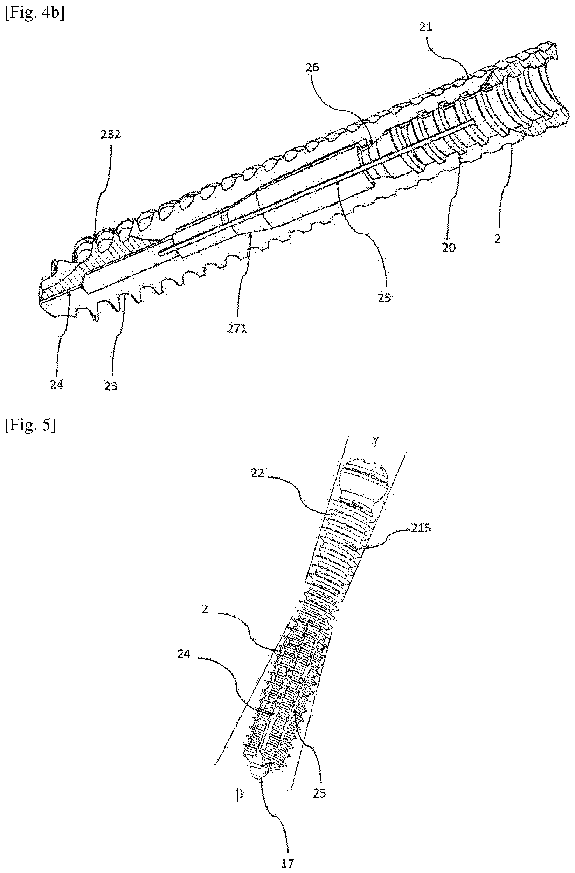

Dans certains modes de réalisation, la portion distale (23) du manchon expansible (2) présente une portion tronconique (291) comprenant un filetage (232) avec une âme conique permettant au manchon expansible (2) de s'enfoncer profondément dans l'os, comme par exemple représenté sur [Fig. 1a] à [Fig. 4b].

Dans certains modes de réalisation, la portion distale (23) du manchon (2) est auto-taraudante et comporte des encoches (231) auto-taraudantes (fraisantes et taraudantes), comme par exemple représenté sur [Fig. 3a] à [Fig. 4b]. Cette portion distale (23) permet de préserver l'os lors de l'implantation tout en évitant de prépercer avant l'insertion de l'implant, et ainsi de conserver une quantité maximale d'os autour de la zone implantée, ce qui améliore la stabilité de l'implant osseux. En effet, le délai d'ostéo-intégration est ainsi réduit ce qui limite la nécessité de rajouter tout type de matériau de comblement osseux, qu'il soit synthétique ou naturel. De plus, la répartition des encoches (231) assure un bon équilibre sur chacune des parties de la portion distale (23) et ainsi une bonne homogénéité de la répartition de l'effort lors de l'insertion de l'implant dans le tissu osseux.

Dans certains modes de réalisation, la vis (1) comprend au moins un marqueur de distance (16), comme par exemple représenté sur [Fig. 2], pour visualiser le moment où le vissage de la vis (1) dans le manchon expansible (2) doit être réalisé dans le sens opposé au vissage du manchon expansible (2) dans le tissu osseux.

Dans certains modes de réalisation, le marqueur de distance (16) est réalisé au laser. Dans certains modes de réalisation, la pose de l'implant comprend les étapes suivantes :
Le vissage de l'implant osseux dans le sens du filetage extérieur (21) jusqu'à ce que le marqueur de distance (16) affleure la surface de la corticale osseuse,
Le vissage de l'implant osseux en vissant dans le sens du second filetage (11) pour compléter le vissage de la vis (1) fileté dans l'os et procéder à l'expansion dudit manchon expansible (2).

Dans certaines variantes de l'invention, l'os cortical est perforé au moyen d'un outil de préforme corticale.

La présente demande concerne également l'expansion d'un implant osseux dans le tissu osseux.

Dans certains modes de réalisation, les profils extérieur de la vis (1) et intérieur du manchon expansible (2) sont complémentaires, de sorte qu'ils fournissent, en configuration expandue :
Un point d'appui proximal supporté par la complémentarité du diamètre externe de la vis (1) avec le diamètre interne du manchon expansible (2),
Un point d'appui distal supporté par la coopération entre le manchon expansible (2) dont le diamètre interne se rétrécit vers la portion distale jusqu'à être inférieur au diamètre externe de la vis (1), et la vis (1),
Un point d'appui « central » localisé entre ces deux points d'appuis, formé par la coopération entre le diamètre externe de la vis (1) et le diamètre interne du manchon expansible (2) qui induisent un diamètre externe du manchon expansible (2) au niveau « central » qui est supérieur au diamètre externe du manchon expansible (2) au niveau du point d'appui proximal.

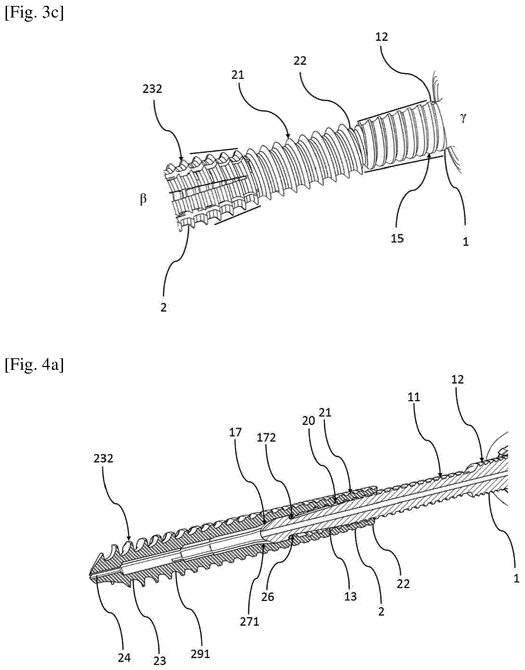

Dans certains modes de réalisation, comme par exemple représenté sur [Fig. 4a] et [Fig. 4b], l'implant est apte à passer d'une position repliée de repos à une position déployée par l'actionnement des filetages inversés en provoquant la pénétration de la vis (1) dans le manchon expansible (2) et en engendrant l'expansion dudit manchon expansible (2) par déformation, grâce au fait que le diamètre extérieur de la vis (1) est supérieur au diamètre intérieur du manchon expansible (2), par au moins un rétrécissement (271) sur une portion distale.

Dans certains modes de réalisation, le rétrécissement (271) est situé, par rapport à la portion proximale et selon l'axe longitudinal (L), à une distance déterminée en fonction de la profondeur, dans le tissu osseux, à laquelle l'expansion est souhaitée. Il est à noter que ladite distance est déterminée par le praticien lui-même notamment au regard de la corticale osseuse et/ou de la compression souhaitée, comme par exemple représenté sur [Fig. 4a] et [Fig. 4b].

Dans certains modes de réalisation, la portion intermédiaire entre l'extrémité proximale et l'extrémité distale du manchon expansible (2) dont le conduit est supérieur au diamètre de l'endroit où se situe le point d'appui central, présente une différence de diamètre.

Dans certains modes de réalisation, le conduit du manchon expansible (2) de la portion intermédiaire entre l'extrémité proximale et l'extrémité distale présente une pente non continue. Ainsi, le rétrécissement (271) peut être situé à une distance variable de l'extrémité proximale pour offrir une expansion à diverses profondeurs, selon le type d'os par exemple.

Dans certains modes de réalisation, la portion proximale de la vis (1) comporte : un filetage externe (15) d'ancrage osseux, comme par exemple représenté sur [Fig. 1a], [Fig. 1b] et [Fig. 2].

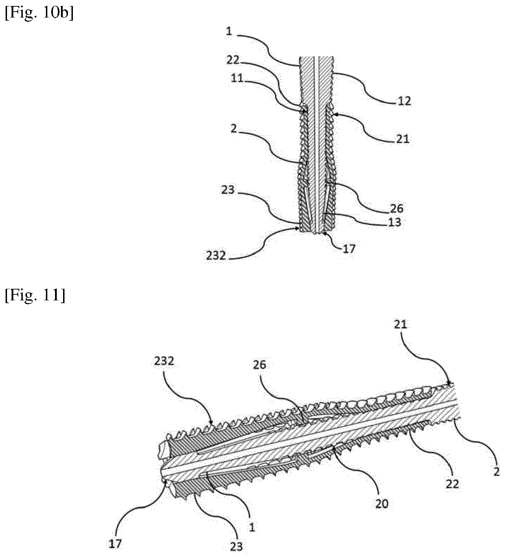

Dans certains modes de réalisation, la vis (1) est configurée pour s'implanter dans le tissu osseux en s'enfonçant à l'intérieur du manchon expansible (2), sa portion proximale comprenant le filetage externe (15) d'ancrage osseux est adaptée à être implantée dans le tissu osseux et présente une portion tronconique dont l'évasement est inversé par rapport à l'évasement de la portion proximale du manchon expansible (2) en position déployée, comme par exemple représenté sur [Fig. 11]. L'inversion de l'évasement des deux troncs de cônes impose une compression et/ou une friction qui améliore la stabilité de l'implant, notamment par le fait que ces deux troncs de cône se trouvent autour de la corticale osseuse.

Dans certains modes de réalisation, la vis (1) est configurée pour s'implanter dans le tissu osseux en s'enfonçant à l'intérieur du manchon expansible (2), sa portion proximale comprenant le filetage externe (15) d'ancrage osseux est adaptée à être implantée dans le tissu osseux et présente une portion cylindrique ou une forme quelconque se déportant.

Le double cône inversé opposé obtenu permet une implantation progressive de l'implant en répartissant les efforts de l'implant osseux dans le tissu osseux lors de l'implantation, permettant la transmission des efforts sur une surface conique et non sur une ligne cylindrique. De plus, le double cône inversé opposé assure un verrouillage axial sur la partie corticale de la vertèbre la plus dense. Il contribue ainsi à l'hyper-stabilité de l'implant osseux dans le tissu osseux.

Dans certains modes de réalisation, comme par exemple représenté sur [Fig. 3a], le manchon expansible (2) présente un angle aigu α à l'extrémité de sa portion distale (23). Cet angle α s'ouvre et augmente au fur et à mesure que la vis (1) pénètre dans le manchon expansible (2), lors de l'expansion.

Dans certains modes de réalisation, comme par exemple représenté sur [Fig. 3b], l'angle α en s'ouvrant de plus en plus lors de l'expansion devient un angle β, l'angle β étant l'angle du manchon expansible (2) expandu.

Dans certains modes de réalisation, comme par exemple représenté sur [Fig. 3c], la partie corticale compresse l'implant osseux et la partie corticale présente un angle γ porté par le manchon expansible (2) ou par la vis (1) ou par les deux. L'angle α en s'ouvrant de plus en plus devient l'angle β du manchon expansible (2) et s'oppose à l'angle γ.

On notera qu'en position déployée, les parois du corps tubulaire (2) peuvent dans certains modes de réalisation être parallèles au lieu de créer un angle β.

Dans certains modes de réalisation, le manchon expansible (2) présente une forme bombée au niveau du point d'appui central, comme par exemple représenté sur [Fig. 5] par la présence des angles α et β.

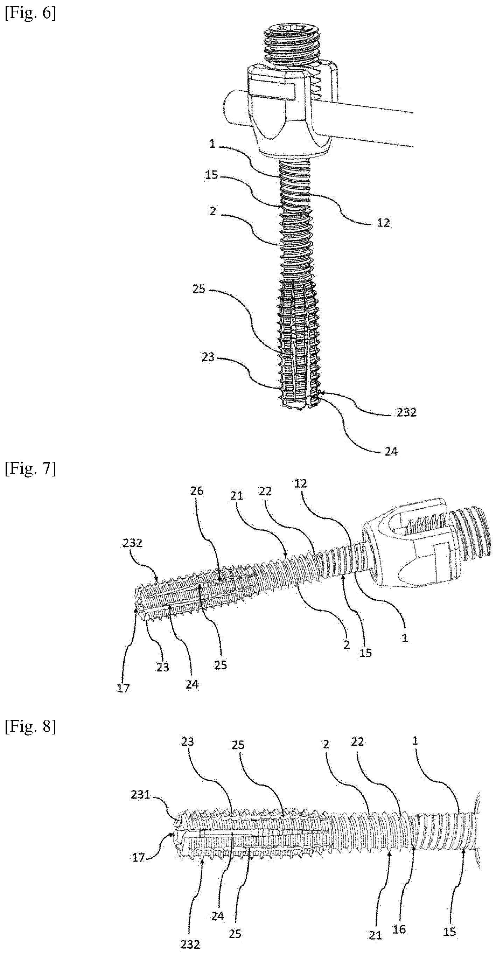

En effet, dans certains modes de réalisation, l'implant est expansible entre, d'une part, une configuration de repos dans laquelle un mécanisme de butée (26) verrouille réciproquement ledit manchon expansible (2) et ledit corps de vis (1) grâce à l'inversion de ces deux pas de vis, comme par exemple représenté sur [Fig. 1a], [Fig. 1b], [Fig. 4a], [Fig. 4b], [Fig. 6], [Fig. 8] à [Fig. 10b] et, d'autre part, une configuration expandue obtenue par l'actionnement desdits filetages complémentaires intérieur et extérieur du manchon expansible (2) et de la vis (1) réciproquement, provoquant la pénétration de la vis (1) dans le manchon expansible (2) et engendrant l'expansion dudit manchon expansible (2), grâce au diamètre extérieur de la vis (1) qui est supérieur au diamètre intérieur du manchon expansible (2), au moins sur une portion distale, par déformation du manchon expansible (2) lors de la pénétration de la vis (1) dans le manchon expansible (2). Dans certains modes de réalisation, en position déployée de l'implant, le second diamètre distal du manchon expansible (2) est supérieur ou égal au premier diamètre proximal du manchon (2), de sorte que le manchon (2) possède une forme cylindrique ou une portion tronconique obtenue par ladite expansion.

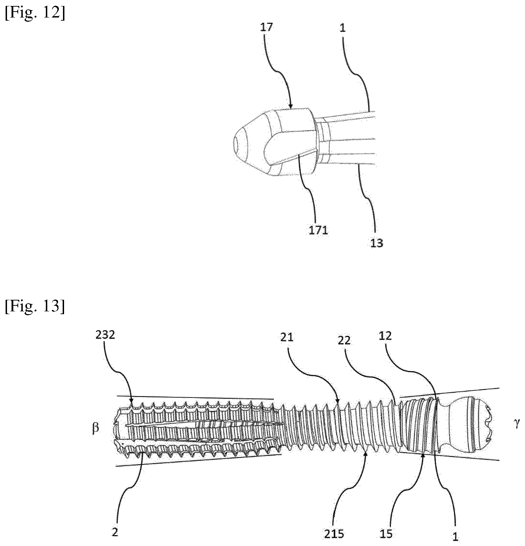

L'implant possède, à proximité de sa portion proximale en position déployée, une portion tronconique proximale s'évasant en direction de la portion proximale et formée :
Soit par le profil extérieur dudit manchon expansible (2), comme par exemple représenté sur [Fig. 3c] dans les modes où l'angle γ est porté par le manchon expansible (2),
Soit par le profil extérieur dudit corps de vis (1), comme par exemple représenté sur [Fig. 5] dans les modes où l'angle γ est porté par la vis (1),
Soit par la complémentarité de forme des profils extérieurs du manchon (2) et du corps de vis (1), en position déployée, comme par exemple représenté sur [Fig. 13] dans les modes où l'angle γ est porté par la vis (1) et le manchon expansible (2). Ladite portion tronconique proximale possédant un filetage externe (15, 215) d'ancrage osseux sur sa périphérie.

Dans certains modes de réalisation, en position déployée de l'implant, le manchon expansible (2) présente une forme tronconique sur au moins une portion proximale (22), comme par exemple représenté sur [Fig. 5] à [Fig. 10b], s'évasant en direction de la portion distale de la vis (1), à proximité de sa portion proximale. Dans certains modes de réalisation, le manchon expansible (2) présente une forme cylindrique ou conique sur au moins une portion proximale (22).

Dans certains modes de réalisation, la vis (1) comprend une pointe (17) sur la pointe de la portion distale (13), comme par exemple représenté sur [Fig. 1a], [Fig. 1b],

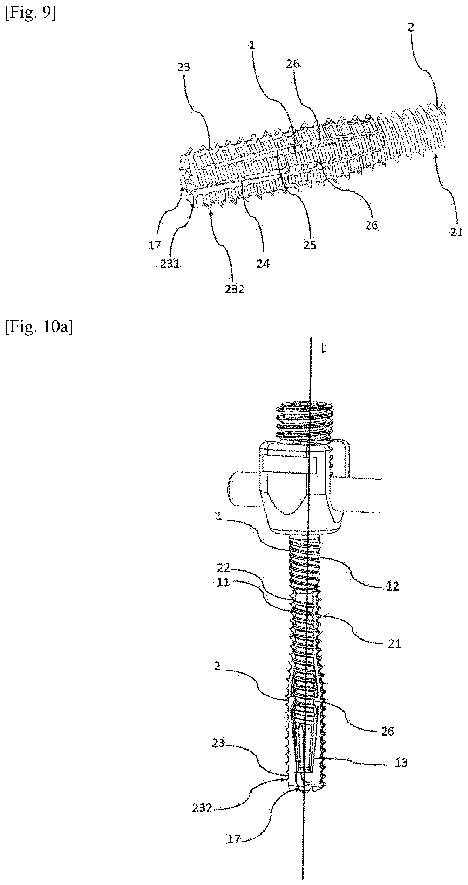

[Fig. 2], [Fig. 5], [Fig. 10a], [Fig. 10b] et [Fig. 12], dont le profil extérieur est complémentaire du profil intérieur de la portion distale (23) du manchon expansible (2).

Dans certains modes de réalisation, ladite portion tronconique de la portion proximale du manchon expansible (2) et ledit profil externe tronconique de la vis (1) sont positionnés bout à bout ou en regard l'un de l'autre et connectés à la portion proximale (22) du manchon expansible (2), l'angle de ladite portion tronconique de la portion proximale du manchon expansible (2) étant supérieur à l'angle dudit profil externe tronconique de la vis (1) pour permettre un évasement plus grand et/ou faciliter l'expansion de l'ensemble.

Dans certains modes de réalisation, comme par exemple représenté sur [Fig. 2], [Fig. 3], [Fig. 5], [Fig. 6], [Fig. 7] et [Fig. 8], le manchon expansible (2) comporte des fentes longitudinales débouchantes (24) s'étendant jusqu'à sa portion distale (23) et des fentes longitudinales non débouchantes (25) permettant l'expansion du manchon expansible (2). Il est préférable qu'il y ait plusieurs fentes débouchantes (24) ou non débouchantes (25), et que la portion distale (23) comporte les deux types de fentes, c'est-à-dire des fentes longitudinales débouchantes (24) et des fentes longitudinales non débouchantes (25).

Dans certains modes de réalisation, la synergie entre les fentes débouchantes (24) et les fentes non débouchantes (25) permet en outre la géométrie de cône tronqué et/ou une forme bombée en position déployée.

Dans certains modes de réalisation, il y a autant d'encoches (231) auto-taraudantes que de fentes longitudinales débouchantes (24).

Dans certains modes de réalisation, les fentes débouchantes (24) et les fentes non débouchantes (25) sont positionnées en décalage l'une par rapport à l'autre sur la longueur du manchon expansible (2). Le décalage des fentes débouchantes (24) et des fentes non débouchantes (25) sur la longueur améliore la souplesse et la résistance mécanique du manchon expansible (2) lors de l'expansion.

Dans certaines variantes de l'invention, une extension de l'ancrage osseux (15, 215) est possible.

Dans certains modes de réalisation, les fentes longitudinales débouchantes (24) et non débouchantes (25) sur la portion distale (23) permettent l'expansion cylindrique du manchon expansible (2). Les fentes longitudinales non débouchantes (25) contribuent à la stabilité de l'implant osseux dans le tissu osseux en permettant lors de l'expansion de pouvoir conserver le profil de contact sur les trois points d'appui entre le manchon expansible (2) et la vis (1), et en laissant les efforts dus à l'expansion se répartir de façon homogène sur la périphérie du manchon expansible (2) expandu. Les fentes longitudinales débouchantes (24) et non débouchantes (25) permettent une expansion radiale de la portion proximale (22) du manchon expansible (2) en respectant la limite élastique de la matière du manchon expansible (2) et son rétreint élastique lors du dévissage.

Dans certains modes de réalisation, lesdites fentes longitudinales débouchantes (24) s'étendent sur 10 à 90 % de la longueur du manchon expansible (2).

Dans certains modes de réalisation, comme cela par exemple est représenté sur [Fig. 3a], la portion du manchon expansible (2) comporte une conicité d'angle alpha (a) en fond de filet assurant l'auto-centrage du manchon expansible (2) dans la cavité réalisée par l'outil de préforme conique anatomique à la forme du manchon expansible (2) lors de l'opération de vissage.

Selon un mode de réalisation alternatif (non représenté) la vis (1) ne comprend pas de partie filetée corticale. Cette variante permet de réaliser un ensemble vis (1) et manchon expansible (2) plus court adapté à d'autre circonstances d'implantations. L'homme du métier comprend que divers types de pointes et de structures peuvent être rapportés à la portion proximale de la vis (1), selon d'autres modes d'assemblage en adaptant la forme de cette portion à cette fin et selon l'objet de l'implant. A titre d'exemples non limitatifs, ces assemblages sont réalisables par vis, clipsage, clavetage, collage, ou soudage.

L'implant osseux proposé dans l'invention peut donc être implanté rapidement et de façon précise dans le tissu osseux, et rester implanté de façon très stable dans le tissu osseux.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un ou plusieurs autre(s) mode(s) de réalisation à moins que l'inverse ne soit explicitement mentionné ou que ces caractéristiques ne soient incompatibles ou que la combinaison ne fonctionne pas.

Plus généralement, des combinaisons de divers types de moyen de retenue de l'implant et/ou de moyen de retenue des épineuses sont envisagées et seront appréciées par l'homme de métier à l'aide des considérations fonctionnelles et structurelles fournies dans la présente demande. De plus, les caractéristiques techniques décrites dans un mode de réalisation donné peuvent être isolées des autres caractéristiques de ce mode à moins que l'inverse ne soit explicitement mentionné, notamment car les considérations fonctionnelles fournies dans la présente demande fourniront une explication suffisante pour que les adaptations structurelles éventuellement nécessaires soient à la portée de l'homme de métier.

Les personnes versées dans l'art, à la lecture de la présente demande, comprendront que des modes de réalisation sous de nombreuses autres formes spécifiques que celles décrites en détail sont possibles sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit être limitée aux détails donnés ci-dessus.

## Revendications

1. Implant d'ancrage osseux à stabilisation corticale apte à être implanté dans un tissu osseux, comprenant :
Un manchon expansible (2) s'étendant entre une portion proximale (22) possédant un premier diamètre intérieur, et une portion distale (23) possédant un second diamètre intérieur inférieur audit premier diamètre intérieur, ces deux portions définissant un axe longitudinal (L) et lesdits premier et second diamètres intérieur définissant un profil intérieur dudit manchon expansible (2), et comprenant, d'une part, au moins un premier filetage (20) à l'intérieur du manchon expansible (2) et, d'autre part, au moins un second filetage (21) à l'extérieur du manchon expansible (2),
Un corps de vis (1) s'étendant entre une portion proximale (12) et une portion distale (13) et présentant, d'une part, le long dudit axe longitudinal (L), un profil extérieur complémentaire au profil intérieur dudit manchon expansible (2) et, d'autre part, au moins un filetage (11) extérieur dont le pas de vis est inversé par rapport audit second filetage extérieur (21) du manchon expansible (2),
L'implant étant apte à passer d'une position repliée de repos à une position déployée par l'actionnement desdits filetages inversés en provoquant la pénétration de la vis (1) dans le manchon expansible (2) et engendrant l'expansion dudit manchon expansible (2) par déformation, grâce au fait que le diamètre extérieur de la vis (1) est supérieur, au moins sur une portion distale, audit second diamètre intérieur du manchon expansible (2), par au moins un rétrécissement (271),
En position déployée de l'implant, le second diamètre intérieur du manchon expansible (2) étant supérieur ou égal au premier diamètre proximal du manchon, Dans lequel
Le manchon possède une forme cylindrique ou une portion tronconique obtenue par ladite expansion,
L'implant possède, à proximité de sa portion proximale en position déployée, une portion tronconique proximale s'évasant en direction de la portion proximale et formée :
Soit par le profil extérieur dudit manchon (2),
Soit par le profil extérieur dudit corps de vis (1),
Soit par la complémentarité de forme des profils extérieurs du manchon (2) et du corps de vis (1), en position déployée,
Ladite portion tronconique proximale possédant un filetage externe (15, 252) d'ancrage osseux sur sa périphérie.

2. Implant selon la revendication 1, **caractérisé en ce que** ledit au moins un rétrécissement (271) est situé, par rapport à la portion proximale et selon l'axe longitudinal (L), à une distance déterminée en fonction de la profondeur, dans le tissu osseux, à laquelle ladite expansion est souhaitée.

3. Implant selon l'une des revendications précédentes, **caractérisé en ce que** la vis (1) est configurée pour s'implanter dans le tissu osseux en s'enfonçant à l'intérieur du manchon expansible (2), sa portion proximale comprenant le filetage externe (15) d'ancrage osseux est adaptée à être implantée dans le tissu osseux et présente un profil externe tronconique sur sa portion proximale dont l'angle d'ouverture du cône est inversé par rapport à celui de la portion tronconique du manchon expansible (2) en position déployée.

4. Implant selon l'une des revendications 1 ou 2, **caractérisé en ce que** la vis (1) est configurée pour s'implanter dans le tissu osseux en s'enfonçant à l'intérieur du manchon expansible (2), sa portion proximale comprenant le filetage externe (15) d'ancrage osseux est adaptée à être implantée dans le tissu osseux et présente un profil externe cylindrique sur sa portion proximale.

5. Implant selon la revendication 3, **caractérisé en ce que** ladite portion tronconique de la portion proximale du manchon expansible (2) et ledit profil externe tronconique de la vis (1) sont positionnés en regard l'un de l'autre.

6. Implant selon la revendication 5, **caractérisé en ce que** l'angle de la portion tronconique de la portion proximale du manchon expansible (2) est supérieur à l'angle du profil externe tronconique de la vis (1) pour permettre un évasement plus grand et améliorer la stabilité primaire.

7. Implant selon la revendication 6, **caractérisé en ce que** la vis (1) comprend au moins un marqueur de distance (16) pour visualiser le moment où le vissage de la vis (1) dans le manchon expansible (2) doit être réalisé dans le sens opposé au vissage du manchon expansible (2) dans le tissu osseux.

8. Implant selon l'une des revendications précédentes, **caractérisé en ce que** la hauteur de filet du second filetage extérieur (21) du manchon expansible (2) est supérieure à celle du filetage mécanique du premier filetage (20) à l'intérieur du manchon expansible (2) et du filetage (11) extérieur de la vis (1).

9. Implant selon l'une des revendications précédentes, **caractérisé en ce que** la portion distale (23) du manchon expansible (2) présente une portion tronconique (291) comprenant un filetage (232) avec une âme conique permettant au manchon expansible (2) de s'enfoncer profondément dans l'os.

10. Implant selon l'une des revendications précédentes, **caractérisé en ce que** la portion distale (23) du manchon expansible (2) comporte des encoches (231) auto-taraudantes.

11. Implant selon l'une des revendications précédentes, **caractérisé en ce que** le manchon expansible (2) comporte des fentes longitudinales débouchantes (24) s'étendant jusqu'à sa portion distale (23).

12. Implant selon la revendication 11 quand celle-ci dépend de la revendication 10, **caractérisé en ce qu'**il y a autant d'encoches (231) auto-taraudantes que de fentes longitudinales débouchantes (24).

13. Implant selon l'une des revendications 1 à 10, **caractérisé en ce que** le manchon expansible (2) comporte des fentes longitudinales non débouchantes (25).

14. Implant selon la revendication 9, **caractérisé en ce que** la vis (1) comprend à sa portion distale (17) une pointe comportant au moins une goujure arrière (171) à bord coupant dont l'angle par rapport à un axe longitudinal (L) définit par les deux extrémités s'étendant entre la portion proximale (22) et la portion distale (23) du manchon expansible (2), est déterminé en fonction du sens de rotation de la vis (1) lors du dévissage depuis la position déployée vers la position de repos, pour fraiser l'os lors de l'extraction de l'implant osseux.

## Patentansprüche

1. Knochenverankerungsimplantat mit kortikaler Stabilisation, das geeignet ist, in ein Knochengewebe implantiert zu werden, umfassend:
eine erweiterungsfähige Hülse (2), die sich zwischen einem proximalen Abschnitt (22), der einen ersten Innendurchmesser besitzt, und einem distalen Abschnitt (23), der einen zweiten Innendurchmesser besitzt, der kleiner als der erste Innendurchmesser ist, erstreckt, wobei diese zwei Abschnitte eine Längsachse (L) definieren und der erste und der zweite Innendurchmesser ein Innenprofil der erweiterungsfähigen Hülse (2) definieren und einerseits mindestens ein erstes Gewinde (20) innerhalb der erweiterungsfähigen Hülse (2) und andererseits mindestens ein zweites Gewinde (21) außerhalb der erweiterungsfähigen Hülse (2) umfassen,
einen Schraubenkörper (1), der sich zwischen einem proximalen Abschnitt (12) und einem distalen Abschnitt (13) erstreckt und einerseits entlang der Längsachse (L) ein Außenprofil, das zu dem Innenprofil der erweiterungsfähigen Hülse (2) komplementär ist, und andererseits mindestens ein Außengewinde (11), dessen Gewindesteigung in Bezug auf das zweite Außengewinde (21) der erweiterungsfähigen Hülse (2) umgekehrt ist, aufweist, wobei das Implantat geeignet ist, sich durch Betätigung der umgekehrten Gewinde von einer eingeklappten Ruheposition in eine ausgeklappte Position zu bewegen, indem das Eindringen der Schraube (1) in die erweiterungsfähige Hülse (2) bewirkt und die Erweiterung der erweiterungsfähigen Hülse (2) durch Verformung verursacht wird, dank der Tatsache, dass der Außendurchmesser der Schraube (1) mindestens an einem distalen Abschnitt größer als der zweite Innendurchmesser der erweiterungsfähigen Hülse (2), durch mindestens eine Verengung (271), ist,
wobei in der ausgeklappten Position des Implantats der zweite Innendurchmesser der erweiterungsfähigen Hülse (2) größer als oder gleich dem ersten proximalen Durchmesser der Hülse ist,
wobei die Hülse eine zylindrische Form oder einen kegelstumpfförmigen Abschnitt, der durch die Erweiterung erhalten wurde, besitzt,
das Implantat in der Nähe seines proximalen Abschnitts in der ausgeklappten Position einen proximalen kegelstumpfförmigen Abschnitt, der sich in Richtung des proximalen Abschnitts aufweitet und ausgebildet wird:
entweder durch das Außenprofil der Hülse (2),
oder durch das Außenprofil des Schraubenkörpers (1),
oder durch die Komplementarität der Form der Außenprofile der Hülse (2) und des Schraubenkörpers (1) in der ausgeklappten Position,
wobei der proximale kegelstumpfförmige Abschnitt an seinem Umfang ein Außengewinde (15, 252) zur Knochenverankerung besitzt.

2. Implantat nach Anspruch 1, **dadurch gekennzeichnet, dass** die mindestens eine Verengung (271) in Bezug auf den proximalen Abschnitt und entlang der Längsachse (L) in einem Abstand angeordnet ist, der in Abhängigkeit von der Tiefe in dem Knochengewebe, bis zu der die Erweiterung gewünscht wird, bestimmt wird.

3. Implantat nach einem der vorstehenden Ansprüche,
**dadurch gekennzeichnet, dass** die Schraube (1) konfiguriert ist, um in das Knochengewebe implantiert zu werden, indem sie innerhalb der erweiterungsfähigen Hülse (2) versenkt wird, ihr proximaler Abschnitt umfassend das Außengewinde (15) zur Knochenverankerung angepasst ist, um in das Knochengewebe implantiert zu werden, und an ihrem proximalen Abschnitt ein kegelstumpfförmiges Außenprofil aufweist, dessen Kegelöffnungswinkel in Bezug auf den des kegelstumpfförmigen Abschnitts der erweiterungsfähigen Hülse (2) in der ausgeklappten Position umgekehrt ist.

4. Implantat nach einem der Ansprüche 1 oder 2,
**dadurch gekennzeichnet, dass** die Schraube (1) konfiguriert ist, um in das Knochengewebe implantiert zu werden, indem sie innerhalb der erweiterungsfähigen Hülse (2) versenkt wird, ihr proximaler Abschnitt umfassend das Außengewinde (15) zur Knochenverankerung angepasst ist, um in das Knochengewebe implantiert zu werden, und an ihrem proximalen Abschnitt ein zylindrisches Außenprofil aufweist.

5. Implantat nach Anspruch 3, **dadurch gekennzeichnet, dass** der kegelstumpfförmige Abschnitt des proximalen Abschnitts der erweiterungsfähigen Hülse (2) und das kegelstumpfförmige Außenprofil der Schraube (1) einander gegenüberliegend positioniert sind.

6. Implantat nach Anspruch 5, **dadurch gekennzeichnet, dass** der Winkel des kegelstumpfförmigen Abschnitts des proximalen Abschnitts der erweiterungsfähigen Hülse (2) größer als der Winkel des kegelstumpfförmigen Außenprofils der Schraube (1) ist, um eine größere Aufweitung zu ermöglichen und die Primärstabilität zu verbessern.

7. Implantat nach Anspruch 6, **dadurch gekennzeichnet, dass** die Schraube (1) mindestens eine Abstandsmarkierung (16) zum Visualisieren des Moments, zu dem das Eindrehen der Schraube (1) in die erweiterungsfähige Hülse (2) in der Richtung entgegengesetzt zu dem Eindrehen der erweiterungsfähigen Hülse (2) in das Knochengewebe erfolgen muss, umfasst.

8. Implantat nach einem der vorstehenden Ansprüche,
**dadurch gekennzeichnet, dass** die Gewindehöhe des zweiten Außengewindes (21) der erweiterungsfähigen Hülse (2) größer als die des mechanischen Gewindes des ersten Gewindes (20) innerhalb der erweiterungsfähigen Hülse (2) und des Außengewindes (11) der Schraube (1) ist.

9. Implantat nach einem der vorstehenden Ansprüche,
**dadurch gekennzeichnet, dass** der distale Abschnitt (23) der erweiterungsfähigen Hülse (2) einen kegelstumpfförmigen Abschnitt (291) aufweist, umfassend ein Gewinde (232) mit einem konischen Kern, der es der erweiterungsfähigen Hülse (2) ermöglicht, tief in den Knochen versenkt zu werden.

10. Implantat nach einem der vorstehenden Ansprüche,
**dadurch gekennzeichnet, dass** der distale Abschnitt (23) der erweiterungsfähigen Hülse (2) selbstschneidende Kerben (231) vorweist.

11. Implantat nach einem der vorstehenden Ansprüche,
**dadurch gekennzeichnet, dass** die erweiterungsfähige Hülse (2) durchgehende Längsschlitze (24) vorweist, die sich bis zu ihrem distalen Abschnitt (23) erstrecken.

12. Implantat nach Anspruch 11, wenn abhängig von Anspruch 10,
**dadurch gekennzeichnet, dass** es ebenso viele selbstschneidende Kerben (231) wie durchgehende Längsschlitze (24) gibt.

13. Implantat nach einem der Ansprüche 1 bis 10,
**dadurch gekennzeichnet, dass** die erweiterungsfähige Hülse (2) nicht durchgehende Längsschlitze (25) vorweist.

14. Implantat nach Anspruch 9, **dadurch gekennzeichnet, dass** die Schraube (1) an ihrem distalen Abschnitt (17) eine Spitze, die mindestens eine hintere Nut (171) mit schneidender Kante vorweist, deren Winkel relativ zu einer Längsachse (L), die durch die zwei Enden definiert ist, die sich zwischen dem proximalen Abschnitt (22) und dem distalen Abschnitt (23) der erweiterungsfähigen Hülse (2) erstrecken, in Abhängigkeit von der Drehrichtung der Schraube (1) bei einem Herausdrehen aus der ausgeklappten Position in die Ruheposition bestimmt wird, um den Knochen bei der Entfernung des Knochenimplantats zu fräsen.

## Claims

1. An osseous anchoring implant with cortical stabilization able to be implanted in an anchoring tissue, comprising:
An expandable sleeve (2) extending between a proximal portion (22) having a first internal diameter, and a distal portion (23) having a second internal diameter smaller than said first internal diameter, these two portions defining a longitudinal axis (L) and said first and second internal diameters defining an internal profile of said expandable sleeve (2), and comprising, on the one hand, at least a first threading (20) inside the expandable sleeve (2) and, on the other hand, at least a second threading (21) outside the expandable sleeve (2),
A screw body (1) extending between a proximal portion (12) and a distal portion (13) and having, on the one hand, along said longitudinal axis (L), an external profile complementary to the internal profile of said expandable sleeve (2) and, on the other hand, at least one external threading (11) whose screw pitch is reversed relative to said second external threading (21) of the expandable sleeve (2),
The implant being able to switch from a folded rest position to a deployed position by the actuation of said reversed threadings by causing the penetration of the screw (1) into the expandable sleeve (2) and generating the expansion of said expandable sleeve (2) by deformation, thanks to the fact that the external diameter of the screw (1) is greater, at least on a distal portion, than said second internal diameter of the expandable sleeve (2), by at least one shrinkage (271),
In the deployed position of the implant, the second internal diameter of the expandable sleeve (2) being greater than or equal to the first proximal diameter of the sleeve,
wherein
The sleeve has a cylindrical shape or a frustoconical portion obtained by said expansion,
The implant has, in the vicinity of its proximal portion in the deployed position, a proximal frustoconical portion flaring towards the proximal portion and formed:
Either by the external profile of said sleeve (2),
or by the external profile of said screw body (1),
or by the shape complementarity of the external profiles of the sleeve (2) and of the screw body (1), in the deployed position,
Said proximal frustoconical portion having an outer osseous anchoring threading (15, 252) over its periphery.

2. The implant according to claim 1, **characterized in that** said at least one shrinkage (271) is located, relative to the proximal portion and along the longitudinal axis (L), at a distance determined as a function of the depth, in the osseous tissue, at which said expansion is desired

3. The implant according to one of the preceding claims, **characterized in that** the screw (1) is configured to be implanted in the osseous tissue by sinking inside the expandable sleeve (2), its proximal portion comprising the outer osseous anchoring threading (15) is configured to be implanted in the osseous tissue and has a frustoconical outer profile on its proximal portion whose cone opening angle is reversed relative to that of the frustoconical portion of the expandable sleeve (2) in the deployed position.

4. The implant according to one of the preceding claims, **characterized in that** the screw (1) is configured to be implanted in the osseous tissue by sinking inside the expandable sleeve (2), its proximal portion comprising the outer osseous anchoring threading (15) is configured to be implanted in the osseous tissue and has an outer cylindrical profile on its proximal portion.

5. The implant according to claim 3, **characterized in that** said frustoconical portion of the proximal portion of the expandable sleeve (2) and said frustoconical outer profile of the screw (1) are positioned facing each other.

6. The implant according to claim 5, **characterized in that** the angle of the frustoconical portion of the proximal portion of the expandable sleeve (2) is greater than the angle of the frustoconical outer profile of the screw (1) to allow greater flaring and improve the primary stability.

7. The implant according to claim 6, **characterized in that** the screw (1) comprises at least one distance marker (16) to visualize the moment when the screwing of the screw (1) in the expandable sleeve (2) must be carried out in the opposite direction to the screwing the expandable sleeve (2) into the osseous tissue.

8. The implant according to one of the preceding claims, **characterized in that** the thread height of the second external threading (21) of the expandable sleeve (2) is greater than that of the mechanical threading of the first threading (20) inside the expandable sleeve (2) and of the external threading (11) of the screw (1).

9. The implant according to one of the preceding claims, **characterized in that** the distal portion (23) of the expandable sleeve (2) has a frustoconical portion (291) comprising a threading (232) with a conical core allowing the expandable sleeve (2) to sink deep into the bone.

10. The implant according to the preceding claims combined, **characterized in that** the distal portion (23) includes self-tapping notches (231).

11. The implant according to one of the preceding claims, **characterized in that** the expandable sleeve (2) includes longitudinal through-slots (24) extending up to the distal portion (23).

12. The implant according to claim 11, when the later depends on claim 10, **characterized in that** there are as many self-tapping notches (231) as there are longitudinal through-slots (24).

13. The implant according to the claims 1 to 10, **characterized in that** the expandable sleeve (2) includes longitudinal non-through slots (25).

14. The implant according to claim 9, **characterized in that** the screw (1) comprises at its distal portion (17) a tip including at least one rear flute (171) with cutting edge whose angle relative to a longitudinal axis (L) defined by the two ends extending between the proximal portion (22) and the distal portion (23) of the expandable sleeve (2), is determined as a function of the direction of rotation of the screw (1) during the unscrewing from the deployed position to the rest position, to mill the bone during the extraction of the osseous implant.
